# EUROPEAN PATENT APPLICATION

(11) **EP 4 316 554 A1**
(43) Date of publication of application: **07.02.2024**
(21) Application number: 22188148.5
(22) Date of filing: 01.08.2022
(51) Int. Cl.: A61M 15/00, G16H 20/13

(54) **METHOD AND SYSTEM FOR MONITORING SPRAY DELIVERY**

(71) Applicant: Apurano Pharmaceuticals GmbH, 83627 Warngau (DE)
(72) Inventor: BRAND, Werner, 83627 Warngau (DE); BLISSE, Marko, 83627 Warngau (DE)
(74) Representative: Maiwald GmbH

(57) **Abstract**

The present disclosure provides a computer-implemented method for monitoring spray delivery of drug-containing liquid for remedying a discomfort. The method comprises a) receiving first input data, the first input data comprising a number of delivered puffs as detected by a counter of a spray device during an intake period, b) receiving second input data comprising user state data representative of self-reported perceived discomfort level, particularly pain level, and/or self-reported perceived side effect severity during the intake period, the user state data being obtained from a user input via a user interface of an application, c) determining, based at least on the first input data and the second input data, a suggested number of delivered puffs for an upcoming intake period, and d) outputting, via the user interface, user instructions indicating the suggested number of delivered puffs.

## Description

### FIELD OF THE INVENTION

The invention pertains to a method for monitoring spray delivery, a data processing system, a spray device, a monitoring system, a use of the data processing system, spray device, and monitoring system, a computer program product, and a computer-readable medium.

### BACKGROUND

At present, methods for monitoring spray delivery of drug-containing liquids are dependent almost entirely on proper handling by a user of a spray device.

Specifically, the user needs to properly apply and document usage of the spray device and report thereon for the purpose of, e.g., determining whether the proper amount of the drug has been delivered, whether the desired effects are achieved by the drug, how any side-effects may correlate with the delivered dosage, and which adjustments could be made to the dosage so as to keep side-effects small while providing high efficacy of the drug.

However, with current systems, it is difficult to ensure reliability, as it hinges on the user's effort and ability.

It is an objective of the present invention to provide a method and systems that aid in overcoming at least some of the above challenges.

### SUMMARY

The object is achieved by the present invention. The invention provides a method for monitoring spray delivery, a data processing system, a spray device, a monitoring system, a use of the data processing system, spray device, and monitoring system, a computer program product, and a computer-readable medium according to the independent claims. Preferred embodiments are laid down in the dependent claims.

The present disclosure provides a computer-implemented method for monitoring spray delivery, and optionally puff sequences, of drug-containing liquid for remedying a discomfort. The method comprises a) receiving first input data, the first input data comprising a number of delivered puffs as detected by a counter of a spray device during an intake period, b) receiving second input data comprising user state data representative of self-reported perceived discomfort level, particularly pain level, and/or self-reported perceived side effect severity during the intake period, the user state data being obtained from a user input via a user interface of an application, c) determining, based at least on the first input data and the second input data, a suggested number of delivered puffs for an upcoming intake period, and d) outputting, via the user interface, user instructions indicating the suggested number of delivered puffs.

In other words, the actual number of delivered puffs delivered over a period of time, i.e., the intake period, which correlates directly with the dosage during said intake period, and information on the perceived efficacy and perceived side effect as reported by a user can be taken as input data for determining how to further proceed in terms of dosage in upcoming intake periods.

The method, particularly the use of first input data that contains data from a counter of a spray device, allows for high reliability that can be made independent from user effort and ability.

For example, if a discomfort level, particularly pain level, is reported to be rather high and the side effect severity is reported to be low during the intake period, the suggested number of puffs for an upcoming intake period may be higher than the number of delivered puffs as detected by the counter during the intake period. As another example, if a discomfort level, particularly pain level, is reported to be very low and the side effect severity is rather high, then the suggested number of puffs for an upcoming intake period may be lower than the number of delivered puffs as detected during the intake period. However, it is not necessary to take into account both, the discomfort level, particularly pain level, and the side-effect severity. As an example, the suggested number of puffs may be adjusted only based on side-effect severity or only based on discomfort level, particularly pain level.

A user may not reliably and precisely document the delivered puffs, such that an adjustment of the above kind would lack precision and reliability if implemented with known systems and methods.

Accordingly, the challenges described above in the context of the known systems and methods are overcome.

A discomfort level, or in other words level of discomfort, is a measure for quantifying a discomfort to be remedied by the spray delivery, specifically, by the drug contained in the drug-containing liquid, i.e. a discomfort targeted by the drug contained in the drug-containing liquid. The discomfort may be associated with or caused by, in particular a symptom of, a disease, in particular a disease to the central nervous system.

For example, a discomfort may be pain, spasticity, tremor, anxiety, insomnia, mania, depression, or the like.

For example a disease may be any pain-causing disease, multiple sclerosis, Parkinson's disease or any other neurodegenerative diseases, depression, borderline syndrome, or any other psychiatry indication.

Spray delivery may entail that a liquid is delivered via a spray nozzle of a spray device. The spray nozzle may be configured to disperse the liquid into a spray, which is delivered to the user. For example spray delivery may be used for delivery of a liquid into a mouth of a user, particularly underneath the tongue or onto the palate, or into a nostril or onto a suitable patch of skin or mucosa of the user.

Spray delivery may be effected by a user activating a trigger, for example, a sprayer head. The activating may be a mechanical actuation of the trigger, e.g., pressing down a sprayer head.

Puffs may also be referred to as a spray burst. A puff may refer to the liquid that is delivered when the trigger is activated once.

The number of puffs, in the present disclosure, may, in particular, be an integer and correspond to a number of trigger activations, particularly full trigger activations.

As an example, in some spray delivery configurations, it may be possible to activate the trigger to its full extent or only to a partial extent, for example when not using it properly. In that case, a puff may correspond to the trigger being activated to the full extent. The result of activating the trigger to a partial extent may be that a partial puff is delivered, which, depending on the embodiment may or may not be counted as a puff for the detected number of delivered puffs.

Moreover, in the context of the present disclosure, puff sequences may be characterized by a pattern of successive puffs, e.g., a number of puffs per delivery time, time between delivery times, overall number of puffs during an intake period, or the like.

According to the present disclosure, a drug-containing liquid may be any liquid in which an active pharmaceutical ingredient (API), e.g., a narcotic API is contained.

According to the present disclosure, receiving the first input data may comprise receiving the first input data at a computing system, in particular from a device external to the computing system, e.g., via a communication interface, the computing system carrying out some or all of the above-described steps. The first data may be received from a counter, which may be incorporated in a spray device.

Receiving the second input data may comprise receiving the second input data at the computing system, in particular, by means of a user input mechanism of the computing system. For example, the second input data may be received from a touch screen and/or touch pad and/or physical buttons.

According to the present disclosure, a user input via a user interface of an application may be any input received by means of a user input mechanism that allows for a user to interact with the user interface of the application. The user interface may comprise a visual interface displayed on a display of the computing system, for example, the mobile device. The user interface may also comprise audio aspects, for example voice input and output. Outputting, via the user interface, the user instructions may comprise an audio and/or visual output.

The application may, for example, be a web application. The application may run on a computing system, for example a mobile device, and the user interface may be displayed on the display of the mobile device.

According to the present disclosure, an intake period may have a predetermined length that depends on the circumstances at hand. For example, an intake period may have such a length that it is expected that in said period of time at least one drug-delivery is suggested. For example, an intake period may be one day long.

According to the present disclosure, an upcoming intake period may, in particular, be the next intake period, i.e., the intake period that directly follows the intake period for which the first data was obtained.

According to the present disclosure, self-reported perceived discomfort level, particularly pain level, may be determined from a user selection on a scale (a numerical scale or descriptive scale) comprising different levels of pain. Alternatively or in addition, self-reported perceived discomfort level, particularly pain level, may be determined from a binary user selection, e.g., tolerable or not tolerable.

According to the present disclosure, a self-reported perceived side effect severity may be determined from a user selection on a scale (a numerical scale or descriptive scale) comprising different levels of severity, either for a single side effect or for a plurality of side-effects collectively or on separate scales, one for each of a plurality of side effects. Alternatively or in addition, self-reported perceived level of severity of side effects may be determined from a binary user selection, e.g., tolerable or not tolerable, for a single side effect or for a plurality of side-effects collectively or for each if a plurality of side effects individually. Alternatively or in addition, self-reported perceived level of severity of side effects may be determined from a selection of a frequency and/or number of side-effect occurrences over the intake period, for one or more side-effects collectively or individually.

According to the present disclosure, determining the suggested number of puffs, based on the first and second input data may comprise determining to maintain, increase, or decrease the number of puffs as compared to the number of puffs comprised in the first input data depending on the second input data, in particular, depending on severity of side-effects and/or discomfort level, particularly pain level. As an example, an increase may be determined when, according to a pre-defined measure, the discomfort level, particularly pain level, is high and the side-effect severity is low. For example, the pre-defined measure may take into account a baseline level and/or baseline severity as provided by the user, e.g., may comprise a scale relative to the baseline level and/or baseline severity. As another example, a decrease may be determined when, according to a pre-defined measure, the discomfort level, particularly pain level, is low and the side-effect severity is high. Other factors discussed below may, however, be taken into account, for example historical data and/or suggested dosage regimens, which may determine a pattern of gradually increasing a dosage that may at least in part offset factors that, taken alone, would indicate a decrease or maintaining of the number off puffs. When multiple factors are taken into account, weighting or prioritizing of said factors may be performed and/or boundary conditions may be set for some of the factors to obtain the suggested number of puffs. The factors, weights, priorities and/or boundary conditions applied for determining the suggested number of puffs are dependent on the specific application at hand, e.g., on the drug and potential risks associated with the drug in terms of addiction, abuse, misuse, or the like.

The method of the present disclosure may comprise repeating steps a) to d) for each of a plurality of intake periods, particularly successive intake periods.

That is, an iterative adjustment of suggested number of delivered puffs may be performed.

In particular, the steps may be repeated until the suggested number of delivered puffs for an upcoming intake period is the same as the detected number of delivered puffs in the intake period at least once or for at least a predetermined number greater than one of consecutive intake periods. That is, the steps may be repeated until it can be assumed with a sufficiently high confidence that a suitable and/or maximum dosage has been reached. If requiring the predetermined number greater than one, it is possible to verify whether the result is stable.

According to the present disclosure the determining may further be based on third input data, the third input data comprising a set of historical data comprising, for each of a/the plurality of intake periods, a sub-set of historical data comprising the first input data and the second input data associated with the intake period and optionally the suggested number of puffs for the intake period. In particular, the method may comprise creating and/or updating the set of historical data.

Each sub-set of the historical data may, for example, allow for determining a detected number of delivered puffs and corresponding discomfort level, particularly pain level, and/or severity of side-effects for a specific intake period. A plurality of such sub-sets may be comprised in the set of historical data, e.g., for a plurality of, particularly successive, intake periods. The set of historical data may be created by storing a first sub-set of historical data, for example. The set of historical data may be updated by adding new sub-sets of historical data, deleting sub-sets of historical data, e.g., deleting older sub-sets and/or adding the most recent sub-set.

The use of historical data may aid in more precisely determining suggestions for the upcoming intake periods. As an example, if a certain number of delivered puffs has, in the past, repeatedly led to undesirable results like high levels of side effects or pain, then this number may be less likely to be suggested than other numbers.

Alternatively or in addition, according to the present disclosure, the determining may further be based on fourth input data, the fourth input data representative of one or more dosage regimens, in particular, wherein the suggested number of delivered puffs may be determined by selecting and optionally adjusting one of the one or more dosage regimens based on the first input data and the second input data and optionally the third input data.

As an example, determining a suggested number of delivered puffs may entail retrieving a dosage regimen that includes a suggested numbers of puffs or a dosage from which said number is derivable.

The selected dosage regimen may be selected based on the at least one of the first to third input data. For example, a dosage regimen may be selected that fits the previously delivered number of puffs (first input data), particularly for more than one intake period. The latter may be determined from the set of historical data.

The selected dosage regimen may optionally be adjusted based at least one of the first to third input data, particularly, a number of puffs suggested by the selected dosage regimen may be increased or reduced depending on the first to third input data. For example, based on the self-reported pain and/or side-effect levels and/or historical data, the suggested number of puffs may be higher or lower than the number of puffs as suggested by the dosage regimen.

Thus, although general guidelines as to dosage of a drug can be generally followed, a quick adjustment based on objective criteria may be performed without consulting a physician.

According to the present disclosure, the first input data comprises, for each puff, a time stamp indicative of the time of delivery of the puff.

Such a time stamp may aid in determining the number of puffs during an intake period, i.e., by determining all puffs having a time stamp within the intake period. This can be done even retrospectively as compared to when only a number of puffs associated with an intake period is determined and stored without associating the individual puffs with a time of administration.

Moreover, it allows for determining patterns within intake periods. Thus, for example, causes for unexpected levels of pain and/or side-effects. As an example, if it is determined that in the course of an intake period the puffs were not applied properly spaced apart, e.g., distributed over a day or a period of days, this may be a cause for unexpected levels of pain and/or side effects. Such analysis may, for example, be useful if the method fails to yield stable results for an extended period of time.

The method of the present disclosure may comprise determining, for at least one of a plurality of intake periods, particularly based on a/the time stamp indicative of the time of delivery of each puff, the number of delivered puffs during the intake period.

In particular, the method may comprise determining an adherence score indicative of adherence to the dosage regimen, based on a difference between the actual number of delivered puffs and a/the suggested number of delivered puffs for the intake period, for example as defined by a dosage regimen.

Generally, adherence to prescribed dosage regimens is difficult to monitor. Based on the adherence score, it may easily be detected when adherence is not sufficient, which may trigger more frequent in-person supervision, for example, and/or allow for analyzing causes for the method failing to yield acceptable results.

According to the present disclosure, the method may comprise determining, based on a sequence and/or frequency of delivered puffs and/or based on a totaled number of delivered puffs within a predetermined period of time, particularly one or more intake periods, that there is an increased risk of misuse and/or abuse and transmitting data indicative of the increased risk to a third-party, e.g., to a/the device accessible to a physician.

In other words, patterns of puff delivery may be used to determine an increased risk of misuse and/or abuse.

The above allows for timely intervention by a third party, e.g., a physician. For example, some patterns may indicate that puffs are not delivered at prescribed intervals, e.g., are clustered too close together or may occur too infrequently or not with a prescribed regularity, which may indicate misuse. Some patterns may indicate that too many puffs are delivered repeatedly, which may indicate abuse.

According to the present disclosure, the method may comprise automatically or semiautomatically creating and/or updating a/the dosage regimen based on at least one of the first to fourth input data, and optionally based on an/the adherence score.

As mentioned above, a dosage regimen may be selected for determining the suggested number of suggested puffs. The method may comprise that such a dosage regimen is created and/or updated. Creating a dosage regimen may, for example, entail that a number of puffs for several upcoming intake periods is assembled. Updating of the dosage regimen may entail that the number of puffs as suggested by a dosage regimen for a plurality of upcoming intake periods is adjusted for at least one of said upcoming intake periods. The first to fourth input data may be used for these steps. For example, the detected number of delivered puffs may be used as a baseline for upcoming intake periods. As another example, if a high level of side-effects is reported, an increase in number of puffs as suggested by the dosage regimen may be performed in smaller steps.

According to the present disclosure, the method may comprise transmitting reporting data to a/the third party, e.g., to a/the device accessible to a/the physician, wherein the reporting data comprises at least one of the first to fourth input data and/or an/the adherence score, and/or data indicative of the increased risk of misuse and/or abuse. In particular, the method may comprise transmitting the reporting data at predetermined times and/or predetermined intervals and/or whenever a data connection is established.

As already mentioned above, providing certain data, such as the reporting data, to a third party allows for timely intervention when there may be a risk of misuse or abuse and may also allow for an analysis of potential causes when the method fails to yield acceptable results.

According to the present disclosure, the first input data may be received at a computing system, in particular a mobile device, from the spray device via a wired or wireless data connection, in particular via Bluetooth or NFC.

The method of the present disclosure may thus be performed in a distributed manner, wherein the counter detecting the number of delivered puffs is incorporated in the spray device, yet the first input data, particularly the detected number of delivered puffs, can (also) be processed at a different device, like a mobile device. This may be advantageous because the technical requirements for the spray device may be kept low, e.g., in terms of memory, CPU, power consumption. Moreover, the second data, i.e., self-reported data, may be received by user input using input means of a device well-equipped for receiving such user input, e.g., the mobile device, such that it may be advantageous to bring together the first and second input data at said computing system, e.g., the mobile device. Finally, many computing systems, particularly a mobile device, will generally be well-equipped to run a suitable application and for communicating with third party devices.

According to the present disclosure, the method may comprise the counter of the spray device detecting the number of delivered puffs and the spray device transmitting the number of delivered puffs as detected by the counter to the computing system, in particular, to a mobile device.

Incorporating the counter in the spray device is advantageous, as a reliable and compact determination of the number of delivered puffs can be more easily ensured than with separate devices and handling by a user does not require particular care or skill. Advantages of transmitting the data to a computing system for further processing have been outlined above.

The present disclosure also provides a data processing system comprising a computing system configured to communicate with a spray device data processing device via a data connection and optionally comprising the spray device data processing device. The data processing system is configured to carry out and/or control the method steps of a method according to the present disclosure, particularly one or more of the method steps outlined above.

That is, a data processing system comprising at least a computing system external to the spray device may be provided and the steps of the present method may be performed at least in part or completely, except for counting the delivered puffs, at said computing system. The computing system may communicate with a data processing device of the spray device, i.e., the spray device data processing device, e.g., to receive the number of delivered puffs. The data processing system of the present disclosure may optionally also comprise the spray device data processing device, which may be configured to determine and transmit the detected number of delivered puffs to the computing system.

The computing system may be fully implemented in a/the mobile device or another single computing device, e.g., a user device. Alternatively, the computing system may be a distributed system, i.e., may be implemented on a plurality of device, which may comprise one or more servers and optionally the mobile device or another user device.

The present disclosure also provides a spray device, particularly configured for use in a method according to the present disclosure, particularly for use for one or more of the method steps outlined above. The spray device according to the present disclosure comprises a counter configured to process sensor data so as to detect a number of delivered puffs and a spray device data processing device, the spray device data processing device configured to transmit the number of delivered puffs to a computing system external to the spray device.

In particular, the spray device data processing device may comprise at least part of a computing logic of the counter.

According to the present disclosure, the counter may be configured such that each delivered puff is counted and/or such that only full puffs are counted.

In particular, may comprise a sensor detecting movement of a trigger element indicative of delivery of a puff. The sensor may, in particular, be a non-contact sensor, for example an optical sensor, or a contact sensor.

For example, depending on proper use and/or configuration of the spray device, a puff may not be fully delivered. The counter may be configured such that also incomplete puffs are counted or such that only full puffs are counted. For example, a sensor may be arranged so as to detect only when a trigger element is fully activated, in which case only full puffs may be counted. Alternatively, a trigger element may be arranged so as to detect when a trigger element is activated to a predetermined degree, e.g., 75 %, and any other trigger events may not be counted.

According to the present disclosure, the counter may be configured to prevent incomplete delivery of a puff. For example, the counter may be configured such that only a full activation of the trigger releases the drug-containing liquid. This protects from ambiguities in the actual dosage of the drug.

According to the present disclosure, the counter may be configured to receive a bottle of liquid to be delivered as a puff, particularly, to at least partially receive the bottle inside a housing. That is, for intended use, the bottle may be arranged in an integrated manner with the counter. This allows for precise counting and compact configuration.

In particular, according to the present disclosure, the counter may be configured to prevent unauthorized removal of the bottle from the counter. In particular, the counter may comprise a locking ring configured to lock the bottle in place and optionally configured such that a special tool is required to unlock the locking ring so as to release the bottle.

This makes allows for a certain degree of tamper proving, thus increasing reliability and risk of abuse and/or misuse. For example, the special tool may only be distributed to a selected group of people and, particularly, may not be provided to the user of the spray device.

Alternatively or in addition, the counter may be configured to detect removal of the bottle from the counter and/or insertion of /the bottle into the counter, and optionally, the spray device configured to transmit an indication and/or count of removal and/or insertion to the computing system. Alternatively or in addition, the counter may be configured to sense the filling level of the bottle, e.g., by means of an optical sensor.

This may provide different advantages. For example, misuse or abuse may be detected based on frequent bottle changes or faster than expected decrease in filling level. As another example, the remaining amount of liquid in the bottle may be estimated based on puffs delivered since changing the bottle and/or based on the change in filling level caused by delivery of a puff. An indication representative of the remaining amount may be output to a user and/or a third party to allow for timely provision of a new bottle. This improves reliability of adherence to a dosage regimen, as the risk of running out of drug is decreased.

The present disclosure also provides a monitoring system comprising the spray device of the present disclosure and a computing system external to the spray device, the monitoring system configured to carry out the steps of method according to the present disclosure, particularly one or more of the method steps outlined above. The computing device may be the computing device as described further above, for example.

The present disclosure also provides a use of the data processing system according to the present disclosure and/or the spray device according to the present disclosure and/or the monitoring system according to the present disclosure for monitoring spray delivery of a drug-containing liquid, particularly for adaptively setting a dosage regimen of the drug and/or detecting a risk of misuse and/or abuse of the drug.

The monitoring may, in particular, comprise monitoring adherence to a dosage regimen, e.g., to suggested number and/or frequency and/or times of delivered puffs, and/or monitoring whether the suggested number of delivered puffs stabilizes over time when repeating the steps of the claimed method and/or yields suggested numbers that can be reasonably expected. Alternatively or in addition, it may comprise monitoring how changes in dosage affect efficacy and/or side-effects, which may allow for improved future selection of dosage regimens.

The present disclosure also provides a computer program comprising instructions which, when the program is executed by a computer, cause the computer to carry out and/or control the steps of any of the methods of the present disclosure.

The present disclosure also provides a computer-readable medium having stored thereon instructions which, when executed by a computer, cause the computer to carry out and/or control the steps of any of the methods of the present disclosure.

The features and advantages outlined above in the context of the method similarly apply to the data processing system, the spray device, a monitoring system, the use of the data processing system, spray device, and monitoring system, the computer program product, and the computer-readable medium described herein.

Further features, examples, and advantages will become apparent from the detailed description making reference to the accompanying drawings.

### BRIEF DESCRIPTION OF THE DRAWINGS

In the accompanying drawings,
Figs. 1a and 1b illustrate schematically different views of a spray device according to the present disclosure;
Figs. 2a and 2b illustrate schematically different views of a spray device according to the present disclosure;
Figs. 3a and 3b illustrate schematically different views of a spray device according to the present disclosure;
Fig. 4 illustrates schematically a data processing system according to the present disclosure;
Fig. 5 illustrates schematically a monitoring system according to the present disclosure; and
Fig. 6 illustrates a method according to the present disclosure.

### DETAILED DESCRIPTION

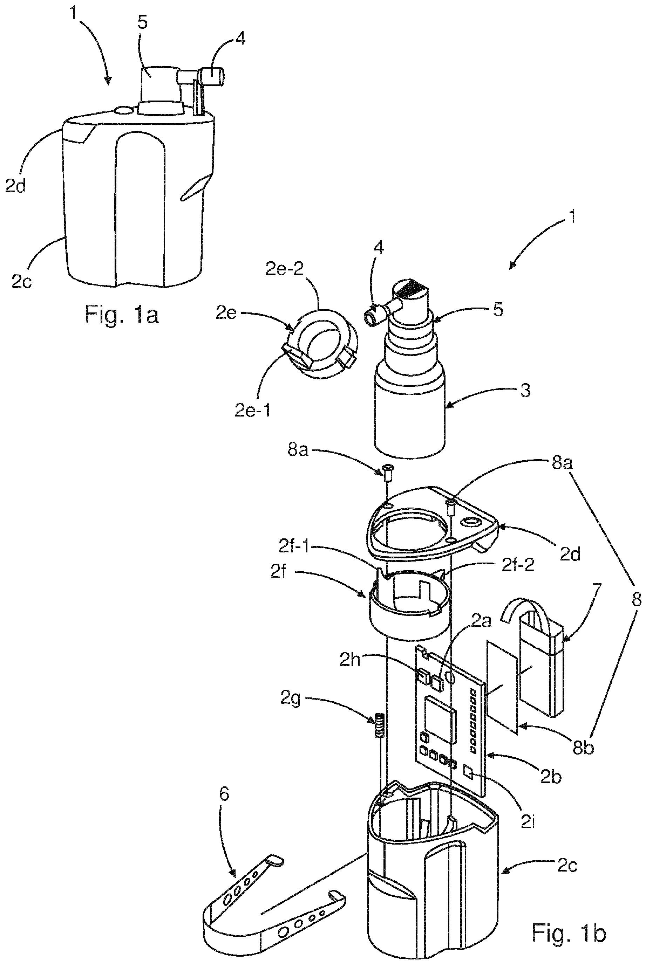

Fig. 1a illustrates schematically and not to scale, a spray device according to the present disclosure. Fig. 1b shows the spray device in a disassembled state.

The spray device 1 shown in Fig. 1 comprises a counter 2, which in this example comprises a sensor 2a, for example a pressure sensor, and computing logic, in this example implemented on a counter PCB 2b. The sensor may be mounted on the PCB. The counter may further comprise a housing comprising housing bottom 2c and a housing cover 2d, and a locking ring 2e. The sensor and the PCB may be arranged inside housing when the spray device is in an assembled state.

The housing bottom may be configured to receive a bottle 3 containing a drug-containing liquid, e.g., a drug-containing nanoformulation, from the bottle 3 via a nozzle 4, e.g. by means of a pumping system 5. The bottle may at least partially be arranged inside the housing, particularly at least partially covered by the housing cover 2d and locked in place by the locking ring 2e. To that end, the locking ring may be configured to mechanically engage with the housing. The locking ring may, for example, be configured such that it requires a special tool 6 for unlocking the locking ring so as to release the bottle, e.g., for changing the bottle. The locking ring comprises a bracket portion 2e-1 extending from a ring portion 2e-2, such that the nozzle's movement is confined by the bracket portion. In particular, the bracket portion may be U-shaped.

The bracket portion may, for example, confine the nozzle's movement in a direction away from the housing and a pivoting movement in a plane perpendicular to said direction.

Shown in Fig. 1b is an exemplary tool, i.e., a pair of tongs, which is not part of the spray device but may be part of the monitoring system 10 according to the present disclosure. The pair of tongs may be configured so as to unlock the locking ring 2e, such that the bottle is released.

The counter may further comprise a trigger element 2f configured to trigger the sensor. In the present example, the trigger element is a trigger ring movably arranged inside the housing except for an extension portion 2f-1 extending through the housing cover. The trigger element is configured and arranged such that delivering a puff, e.g. by operating the pumping mechanism, operates the trigger element. In the present example, operating the pumping mechanism pushes the trigger element from via the extension portion of the trigger element. The trigger element may thus be pushed from a resting position to a trigger position.

The trigger element may be configured such that it triggers the sensor upon reaching the trigger position. For example, protrusion 2f-2 may be configured to trigger the sensor. The trigger element may be spring-loaded when in the trigger position so as to return to a resting position. An exemplary spring 2g for spring-loading the trigger element is shown in Fig. 1b. In Fig. 1b, as an example, the trigger element and housing bottom are shaped in such a manner that the housing bottom guides the movement of the trigger element, e.g., by one or more pairs of a groove and a protrusion engaging with each other.

The spray device may further comprise an energy source 7, e.g. a battery, configured to power the computing logic on the PCB and optionally other electronics. The spray device may further comprise fixing elements 8, for example first fixing elements 8a, like screws, fixing the housing cover to the housing bottom, and/or second fixing elements 8b, for example adhesive tape, for fixing the energy source, e.g., battery. For example, the energy source may be fixed to the PCB.

Optionally, the counter may comprise a sensor 2h for detecting presence and/or absence of a bottle inside the housing. For example, the signals from sensor 2h may be used for tracking changes of the bottle and/or for issuing a warning and/or an error signal when a bottle is missing.

The counter may also comprise one or more wireless communication interfaces 2i, for example at least one of Bluetooth interface, an NFC interface, a mobile communication interface, and a WiFi interface or the like, for transmitting data to an external computing system.

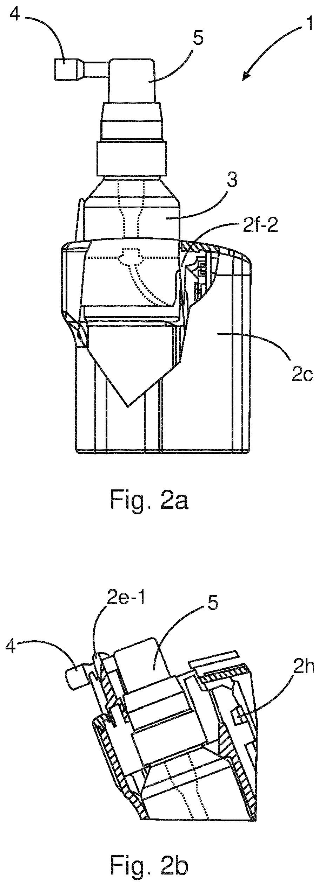

Figs. 2a and 2b show different views of at least part of a spray device according to the present disclosure, e.g., as shown in Figs. 1a and 1b. In Fig. 2a the bottle is not fully inserted in the housing.

In Fig. 2b, the sensor 2h for detecting the presence and/or absence of a bottle and the bracket confining the movement of the nozzle are shown in more detail.

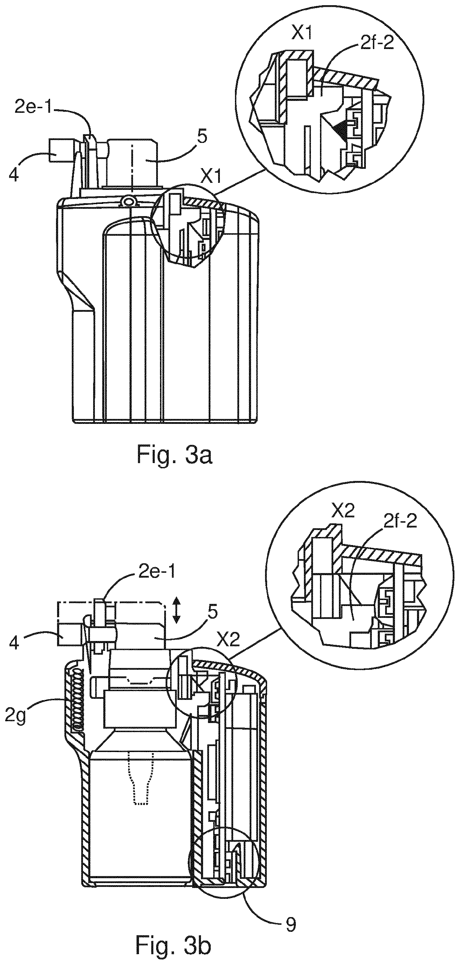

In Figs 3a and 3b, a spray device according to the present disclosure, e.g. as shown in Figs. 1a, 1b, 2a, or 2b, is shown in two different states. In the first state, shown in Fig. 3a, the trigger element is in a resting position. In the second state, shown in Fig. 3b, the trigger element is in a trigger position, springloaded by a spring so as to return to a resting position. It can be seen that the locking ring, particularly its bracket, is configured such that, while confining the movement of the nozzle, it allows the nozzle to move downwards when the pumping system is operated to deliver a puff and, at the same time, thereby causing the trigger element to move from the resting position to the trigger position. The respective position of the protrusion 2f-2 configured to trigger the sensor 2a of the counter is also shown.

In Fig. 3b, an optional port 9 for a wired data connection and/or power supply, for example a USB-A port, USB-C port, or the like, is shown.

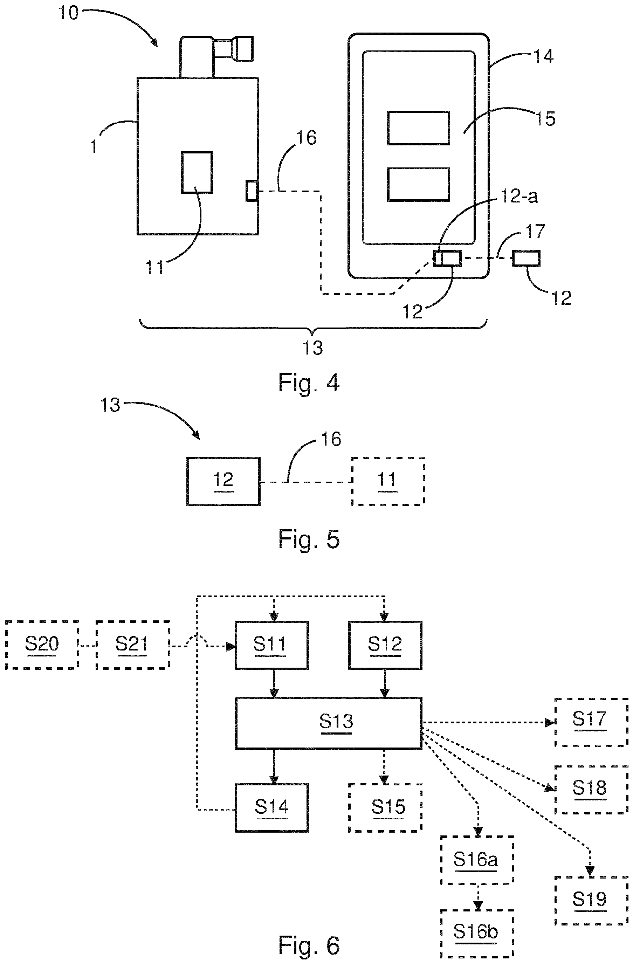

Fig. 4 illustrates an exemplary monitoring system 10 according to the present disclosure.

The monitoring system comprises a spray device according to the present disclosure, e.g., as shown above. The spray device may comprise a spray device data processing device 11, implemented, for example, on the PCB. The data processing device may comprise the above-described computing logic of the counter. The data processing device may be configured to process data from sensors so as to count the number of puffs and/or to apply a time stamp to each puff and/or so as to detect the presence of a bottle. It may also be configured to perform other types of processing, for example, determine cumulative number of puffs having occurred over a predetermined time-period, e.g., during a predetermined intake period and/or from the time of changing the bottle. Furthermore, the data processing device may be configured to transmit at least some of said data, particularly the number of puffs, to external computing systems, e.g. computing system 12 described below.

The data processing device 11 may also be configured to prompt a display device, e.g., incorporated into the housing of the counter, to display a representation of a number of puffs and/or a filling level of the bottle and/or projected remaining time until the bottle requires changing.

The monitoring system also comprises a computing system 12 external to the spray device. The computing system may be configured to carry some or all of the method steps of the present disclosure, with the exception of steps that can only be carried out by the spray device, e.g., obtaining the sensor data to be used for detecting the number of puffs and/or presence of a bottle and/or sensing a filling level in the bottle. In particular, the computing system may be configured to, based on the detected number of puffs and other input data, particularly the second input data, determine the suggested number of puffs for the upcoming intake period. It may, in particular, be configured to process user input data and/or data received from other devices.

In the example shown in Fig. 4, at least part of the computing system is implemented on a mobile device 14. Optionally, the entire computing system 12 may be implemented on the mobile device, or the computing system may be a distributed system and only part of it may be implemented on the mobile device. Another part may, for example, be implemented on a server connected with the mobile device via a data connection 17. A user interface 15, e.g. of an application, e.g., a web application, may be rendered on the mobile device.

The spray device data processing device 11 and the computing system 12 of this example are configured to communicate via a data connection 16, for example a wireless connection like Bluetooth, NFC, mobile communication network, or WiFi. The connection may be established via the spray device's interface 2i described above and a communication interface 12a of the computing system 12. In particular, the detected number of puffs and optionally additional data, e.g., timestamps, may be provided from the data processing device 11 to the computing system 12 via the data connection.

Fig. 5 illustrates a data processing system 13 according to the present disclosure, which comprises a computing system 12 (distributed or non-distributed), for example the computing system as described in the context of Fig. 4 and optionally a spray device data processing device, for example the spray device data processing device as shown in Fig. 4.

According to the present disclosure, a computer-implemented method for monitoring spray delivery of drug-containing liquid is provided. An exemplary method is illustrated in Fig. 6.

In step S11, first input data are received, the first input data comprising a number of delivered puffs as detected by a counter of a spray device during an intake period.

In step S12, second input data are received, the second input data comprising user state data representative of self-reported perceived discomfort level and/or self-reported perceived side effect severity during the intake period, the user state data being obtained from a user input via a user interface of an application.

Steps S11 and S12 may be performed simultaneously or subsequently.

In step S13, a suggested number of delivered puffs for an upcoming intake period is determined based at least on the first input data and the second input data. Thus, step S13 may be performed subsequently to steps S11 and S12.

In step S14, subsequently to step S13, user instructions indicating the suggested number of delivered puffs is output via the user interface.

In optional step S15, a set of historical data is created and/or updated, the set of historical data comprising, for each of a/the plurality of intake periods, the first input data and the second input data associated with the intake period and optionally the suggested number of puffs for the intake period.

In optional step S16a, for at least one of a plurality of intake periods, particularly based on a/the time stamp indicative of the time of delivery of each puff, the number of delivered puffs during the intake period is determined.

In optional step S16b, an adherence score indicative of adherence to the dosage regimen is determined, based on a difference between the actual number of delivered puffs and a/the suggested number of delivered puffs for the intake period, for example as defined by a dosage regimen. For example, step S16b may be performed after step S16a.

In optional step S17, based on a sequence and/or frequency of delivered puffs and/or based on a totaled number of delivered puffs within a predetermined period of time, particularly one or more intake periods, it is determined that there is an increased risk of misuse and/or abuse and optionally data indicative of the increased risk is transmitted to a third-party, e.g., to a/the device accessible to a physician. This step may be performed at any time after step S13.

In optional step S18, a/the dosage regimen based on at least one of the first to fourth input data, and optionally based on an/the adherence score is automatically or semiautomatically created and/or updated. This step may be performed at any time after step S13.

In optional step S19, reporting data may be transmitted to a third party, e.g., to a device accessible to a/the physician, for example to report an adherence score, and/or data indicative of increased risk of misuse and/or abuse.

In optional step S20, a counter of a spray device, e.g. the counter 2 described above, detects the number of delivered puffs. This step, accordingly, may be performed prior to step S11.

In subsequent optional step S21, the detected number of puffs is transmitted to a computing system external to the spray device, e.g. the computing system 12 described above. This step may be performed after step S20 and prior to step S11, for example.

After step S14, the method may return to steps S11 and S12 and subsequent steps S13 and S14, particularly repeated once of each of a plurality of intake periods. That is, the suggested number of puffs may be repeatedly checked and potentially adjusted for each intake period.

Further examples and embodiments will be explained below.

The monitoring system 10 described above may also be referred to as an adaptive titration tool. As seen above, it comprises two main components, a counter of a spray device and a computing system, e.g. computing system 12 that uses the data received from the counter as input for performing some of the method steps of the method of the present disclosure. In practice, an application, e.g. a web application, may implement at least some of these method steps. The application may thus provide functionality for an adaptive titration, for example of a narcotic drug (narcotic agent) against chronic pain delivered by means of the spray device. That is, in the present example, pain is used as an example for the claimed discomfort. However, the example can similarly be applied to other types of discomforts according to the present disclosure. Their quantification can be done in any suitable manner, particularly, no standardize measure is required.

As explained above, a counter according to the present disclosure may electronically detect puffs, e.g. by the stroke of a spray head, via a sensor in the housing of the counter, the stroke indicative of a puff having been triggered. This signal may be stored in the internal memory and wirelessly transmitted to the web application, e.g., via Bluetooth. The counter may also be configured record the change of the bottle, for example, in order to thus display a filling level indication based on a number of puffs delivered after the change of the bottle.

An exemplary method for adaptive titration of a narcotic agent against chronic pain will be described below.

An algorithm for an adaptive titration (in short "adaptive titration tool") may be used for implementing the method of the present disclosure, e.g., for use in an individual titration of a spray against chronic pain with a narcotic drug, e.g. with the active substance AP707.

The adaptive titration tool adaptively adjusts a user to the optimal dose at which the user experiences the least possible side effects from administration of the narcotic drug. In addition, the adjustment phase determines the dose at which patients achieve optimal reduction in their chronic pain.

The titration regimen may, in this example, be divided into two phases, the initial phase, which is identical for all patients, and the individual adaptive titration phase.

After the, e.g. four-day, initial phase, an algorithm is used to evaluate the titration based on one or more of the following parameters (second input data) to determine the next dose (i.e., the suggested number of delivered puffs for an upcoming intake period):
- Self-reported rating of level of severity of the side effects, e.g., via an application interface, e.g., one of vomiting, prolonged tachycardia, severe diarrhea, severe dizziness, hallucinations
- Pain score, e.g., NRS, wherein, for example, 0=no pain and 10= worst pain imaginable
- Pain tolerability (yes, no)

The data may be obtained, for example, by means of a questionnaire. The parameters may be prioritized in terms of their weight when determining the next dose.

The titration questionnaire may, for example, be filled out daily by the user via the application. For example, this may be done in the evening and an evaluation may take place afterwards, e.g., during the night. The user may then be shown the dosage (number of puffs) for the respective intake times of the following day in the user interface of the application.

As an example, after the initial phase, an evaluation and suggestion of a dosage may be performed as follows:
If side effects occur, no increase of dosage is suggested.

If no side effects occur, the pain NRS is evaluated. If the daily pain NRS is less than the baseline pain NRS, the dose is increased by an integer multiple (n > 0) of one puff, e.g. by exactly one puff. Otherwise, there is a dose increase by an integer multiple m (m>n), for example, by two puffs.

When increasing the dose, the number of puffs may be increased, for example, according to the scheme of the initial phase.

As an example, for increasing the dosage by one puff a day (intake period) and each day has more than one intake time, one puff may be added for one of the intake times on each of a plurality of successive days until the number of puffs has been increased by one puff for each intake time. Each day one puff may be added to a different intake time. For example, when there are first to fourth intake times (in that chronological order), then on the first day one puff may be added at the third intake time, on the second day one puff may be added at the first intake time, on the third day one puff may be added at the fourth intake time, and on the fourth day one puff may be added at the second intake time. The above-described pattern of adding one puff for one of the intake times on each of a plurality of successive days may be repeated. Similarly, when increasing the dosage by more than one puff a day (intake period) and the day has more than one intake time, one puff may be added for each of a subset of intake times, for example, on each of a plurality of successive days until the number of puffs has been increased for each intake time. The subsets may be non-overlapping for successive days. For example, on one day, one puff may be added for the first and one for the third intake time, and on the next day, one puff may be added for the second and one for the fourth intake time. For example, this may be applied on a fifth and sixth day after the above pattern for the first to fourth day has been carried out once or on even later days after the above pattern for the first to fourth days has been carried out repeatedly. This pattern may also be repeated. If no further increase of the dosage is suggested, e.g., due to side effect severity, the latest dosage and intake times may be maintained. In the above example with four days, for example, if no increase is suggested after the fourth day, the dosage of the fourth day at the intake times of the fourth day may be maintained. This may similarly be the case on the other days of the above examples.

There may be upper limits of overall duration of the adaptive titration, e.g., a maximum duration of four weeks. The exact duration may be individualized for the respective user. Once a specific patient specific dose limit is reached, the last dose may be maintained, e.g., until a physician creates a follow-up prescription, for example in the application.

Potential endpoints for dose determination may be:
- a daily NRS below average baseline NRS, e.g., daily NRS ≤70% of average baseline, is reached
- a first number of puffs, e.g., eight puffs, is reached and the pain tolerability is Yes and the daily NRS ≤40% of average baseline NRS.
- a second number of puffs, e.g., twelve puffs, is reached and the pain tolerability is Yes and the daily NRS ≤40% of average baseline NRS
- a third number of puffs, e.g. 16 puffs, per intake period, e.g., per day, is reached.

Optionally, an additional integrated safety mechanism reducing risk for the user may be provided. For example, when a predetermined level of severity of certain side effects or other adverse effects is exceeded once and/or during each of a predetermined number of consecutive intake periods (e.g., number of days), the dose may be set to zero or reduced by a predetermined amount and/or a physician consultation may be recommended to the user. Optionally, a physician may automatically be alerted.

It is to be understood that, while pain is one of the discomforts described in detail in the above example, this is only one of many examples of potential discomforts.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

## Claims

1. A computer-implemented method for monitoring spray delivery of drug-containing liquid for remedying a discomfort, the method comprising
a) receiving (S11) first input data, the first input data comprising a number of delivered puffs as detected by a counter (2) of a spray device(1) during an intake period,
b) receiving (S12) second input data comprising user state data representative of self-reported perceived discomfort level and/or self-reported perceived side effect severity during the intake period, the user state data being obtained from a user input via a user interface (15) of an application,
c) determining (S13), based at least on the first input data and the second input data, a suggested number of delivered puffs for an upcoming intake period, and
d) outputting (S14), via the user interface (15), user instructions indicating the suggested number of delivered puffs.

2. The method of claim 1 comprising repeating steps a) to d) for each of a plurality of intake periods, particularly successive intake periods.

3. The method of any of the preceding claims,
wherein the determining is further based on third input data, the third input data comprising a set of historical data comprising, for each of a/the plurality of intake periods, a sub-set of historical data comprising the first input data and the second input data associated with the intake period and optionally the suggested number of puffs for the intake period, in particular, wherein the method comprises creating and/or updating (S15) the set of historical data; and/or
wherein the determining is further based on fourth input data, the fourth input data representative of one or more dosage regimens, in particular, wherein the suggested number of delivered puffs is determined by selecting and optionally adjusting one of the one or more dosage regimens based on the first input data and the second input data and optionally the third input data.

4. The method of any of the preceding claims,
wherein the first input data comprises, for each puff, a time stamp indicative of the time of delivery of the puff, and/or
wherein the method comprises determining (S16a), for at least one of a plurality of intake periods, particularly based on a/the time stamp indicative of the time of delivery of each puff, the number of delivered puffs during the intake period,
in particular, wherein the method comprises determining (S16b) an adherence score indicative of adherence to the dosage regimen, based on a difference between the actual number of delivered puffs and a/the suggested number of delivered puffs for the intake period, for example as defined by a dosage regimen.

5. The method of any of the preceding claims, comprising determining (S17), based on a sequence and/or frequency of delivered puffs and/or based on a totaled number of delivered puffs within a predetermined period of time, particularly one or more intake periods, that there is an increased risk of misuse and/or abuse and transmitting data indicative of the increased risk to a third-party, e.g., to a/the device accessible to a physician.

6. The method of any of the preceding claims comprising automatically or semiautomatically creating and/or updating (S18) a/the dosage regimen based on at least one of the first to fourth input data, and optionally based on an/the adherence score.

7. The method of any of the preceding claims, comprising transmitting (S19) reporting data to a/the third party, e.g., to a/the device accessible to a/the physician, wherein the reporting data comprises at least one of the first to fourth input data and/or an/the adherence score, and/or data indicative of the increased risk of misuse and/or abuse, in particular transmitting the reporting data at predetermined times and/or predetermined intervals and/or whenever a data connection is established.

8. The method of any of the preceding claims, wherein the first input data is received at a computing system, in particular a mobile device, from the spray device via a wired or wireless data connection, in particular via Bluetooth or NFC.

9. The method of any of the preceding claims, further comprising the counter of the spray device detecting (S20) the number of delivered puffs and the spray device transmitting (S21) the number of delivered puffs as detected by the counter to the computing system (12), in particular, to a mobile device (14).

10. A data processing system (13) comprising a computing system (12) configured to communicate with a spray device data processing device (11) via a data connection (16) and optionally comprising the spray device data processing device (11), the data processing system (13) configured to carry out and/or control the method steps of any one of claims 1 to 9.

11. A spray device (1), particularly configured for use in a method according to any one of claims 1 to 9, the spray device comprising
a counter (2) configured to process sensor data so as to detect a number of delivered puffs;
a spray device data processing device (11), the spray device data processing device (11) configured to transmit the number of delivered puffs to a computing system (12) external to the spray device (1),
in particular, wherein the spray device data processing device (11) comprises at least part of a computing logic of the counter (2).

12. The spray device (1) of claim 11,
wherein the counter (2) is configured such that each delivered puff is counted and/or such that only full puffs are counted, in particular, comprises a sensor detecting movement of a trigger element indicative of delivery of a puff; and/or
wherein the counter (2) is configured to prevent incomplete delivery of a puff; and/or wherein the counter (2) is configured to receive a bottle (3) of liquid to be delivered as a puff, particularly, to at least partially receive the bottle (3) inside a housing, in particular,
wherein the counter (2) is configured to prevent unauthorized removal of the bottle (3) from the counter (2), in particular, comprises a locking ring (2e) configured to lock the bottle (3) in place and optionally configured such that a special tool is required to unlock the locking ring (2e) so as to release the bottle (3), and/or
wherein the counter (2) is configured to detect removal of the bottle (3) from the counter (2) and/or insertion of /the bottle (3) into the counter (2), and optionally, the spray device (1) configured to transmit an indication and/or count of removal and/or insertion to the computing system (12).

13. A monitoring system (10) comprising the spray device (1) of claims 11 or 12 and a computing system (12) external to the spray device (1), the monitoring system (10) configured to carry out the steps of any one of claims 1 to 9.

14. Use of the data processing system (13) according to claim 10 and/or the spray device (1) according to claim 11 or 12 and/or the monitoring system (10) according to claim 13 for monitoring spray delivery of a drug-containing liquid, particularly for adaptively setting a dosage regimen of the drug and/or detecting a risk of misuse and/or abuse of the drug.

15. A computer program comprising instructions which, when the program is executed by a computer, cause the computer to carry out and/or control the steps of the method of any one of claims 1 to 9, or a computer-readable medium having stored thereon instructions which, when the program is executed by a computer, cause the computer to carry out and/or control the steps of the method of any one of claims 1 to 9.
